# EUROPÄISCHE PATENTSCHRIFT

(11) **EP 3 232 988 B1**
(45) Veröffentlichungstag und Bekanntmachung des Hinweises auf die Patenterteilung: **28.07.2021**
(21) Anmeldenummer: 15813415.5
(22) Anmeldetag: 17.12.2015
(51) Int. Cl.: A61F 2/16

(54) **INTRAOKULARLINSE MIT IM OPTISCHEN TEIL INTEGRIERTEN EINLINSEN-TELESKOP**
INTRAOCULAR LENS WITH SINGLE LENS TELESCOPE INTEGRATED IN THE OPTICAL PART THEREOF
LENTILLE INTRAOCULAIRE POURVUE D'UN TÉLESCOPE À LENTILLE UNIQUE INTÉGRÉ DANS L'ÉLÉMENT OPTIQUE

(30) Priorität: 18.12.2014 DE 102014119010
(43) Veröffentlichungstag der Anmeldung: 25.10.2017
(73) Patentinhaber: Carl Zeiss Meditec AG, 07745 Jena (DE)
(72) Erfinder: FIALA, Werner, 1180 Wien (AT); GERLACH, Mario, 16548 Glienicke-Nordbahn (DE)
(74) Vertreter: Hofstetter, Schurack & Partner
(86) Internationale Anmeldenummer: PCT/EP2015/080314
(87) Internationale Veröffentlichungsnummer: WO 2016/097192

(56) Entgegenhaltungen:
- EP-A1- 1 818 023
- EP-A2- 0 897 702
- US-A- 4 666 446
- US-A- 5 391 202
- US-A- 5 814 103
- US-B1- 8 579 970

## Beschreibung

Die Erfindung betrifft eine Intraokularlinse mit einem optischen Teil und einem daran anschließenden haptischen Teil, wobei der optische Teil ein optisch abbildendes Element und ein Teleskop aufweist.

### Stand der Technik

Das menschliche Auge kann aufgrund seines relativ komplexen Aufbaus und somit auch insbesondere der zur optischen Abbildung vorgesehenen Teile und/oder anderweitiger Einflussfaktoren mit unterschiedlichsten Sehfehlern behaftet sein. Diese können individuell in der Stärke unterschiedlich ausgeprägt sein, andererseits kann auch eine Mehrzahl unterschiedlicher Sehfehler in einem Auge vorliegen.

Augenlinsen zur Korrektur von Sehfehlern sind in Form von Intraokularlinsen in vielfältigsten Ausgestaltungen bekannt.

Eine derartige Intraokularlinse ist beispielsweise aus der DE 694 31 428 T2 bekannt. Mit Intraokularlinsen, die ein Teleskop aufweisen, ist ein Sichtfeld eines Patienten erweitert, der an einer Degeneration der Makula leidet. Bei einer Makuladegeneration sind die betroffenen Personen nicht in der Lage, ohne spezielle teleskopische oder mikroskopische Gläser zu lesen, welche eine Vergrößerung des Objekts auf der Retina und somit der Makula erzeugen.

Bei der bekannten Intraokularlinse ist der Aufbau jedoch äußerst komplex und die Intraokularlinse ist schwierig herzustellen. Denn die Intraokularlinse benötigt mehrere separate Teile, und zwar einerseits ein Körperelement, das konvex-konvex oder konvexplanar ausgestaltet sein kann. Zentral mittig weist dieses Körperelement eine durchgehende Bohrung auf, in welcher separate und beabstandet zueinander angeordnete Linsenelemente des Teleskops positioniert sind. Es kann vorgesehen sein, dass ein Linsenelement einstückig mit dem Körperelement ausgebildet ist. Das zweite Linsenelement muss jedoch zwangsweise stets als separates Teil bereitgestellt werden, da in einem Hohlraum zwischen den beiden Linsenelementen eine Gasfüllung eingebracht werden muss und auch zwangsweise ein derartiger Hohlraum ausgebildet werden muss. Durch diese Ausgestaltung ist die Positionierung der beiden konvexen Linsen zueinander äußerst schwierig und dauerhaft positionssicher nicht möglich. Dadurch können sich unerwünschte Abbildungseigenschaften des aus mehreren separaten Linsen ausgebildeten Teleskops ergeben und somit kann die Intraokularlinse in ihrer spezifischen Funktionalität zur Sichtweitenanpassung bei einem Patienten mit einer Makuladegeneration nur eingeschränkt Verbesserung bringen.

Eine Intraokularlinse, die ebenfalls ein Teleskop aufweist, ist aus der DE 11 2010 004 191 T5 bekannt. Auch dort sind zwei separate Linsenelemente zum Aufbau des Teleskops zwingend vorgesehen, die über Verbindungselemente verbunden sind, um eine Relativbewegbarkeit der zwei Linsenelemente des Teleskops ermöglichen zu können. Auch diese Ausgestaltung ist äußerst komplex im Aufbau und schwierig herzustellen, sodass auch hier eine eingeschränkte Funktionalität im Hinblick auf die Sichtfeldgestaltung für einen Patienten mit einer Makuladegeneration auftritt.

Des Weiteren ist aus der DE 699 21 648 T2 eine Intraokularlinse mit einem schwenkbaren Fernrohr bekannt. Der dortige Aufbau übersteigt im Hinblick auf die Komplexität die Ausführungen, wie sie in den beiden oben genannten Dokumenten zum Stand der Technik genannt sind.

Ferner ist aus der US 8 579 970 B1 eine Intraokularlinse bekannt, die aus einem teleskopisch optischen Teil besteht, wobei dieses Teleskop eine Mehrzahl von Gaslinsen und einen Mehrzahl von Festkörperlinsen aufweist, die entlang der optischen Achse in Reihe zueinander ausgebildet sind.

Aus der EP 0 897 702 A2 ist ein Intraokularlinsenimplantat mit einem optischen Element und einem Teleskop bekannt.

Bei allen Ausführungen ist darüber hinaus die jeweilige Dicke, insbesondere die Mittendicke der Intraokularlinse, sehr groß, und aufgrund der Ausgestaltung ist die Intraokularlinse als solche relativ steif. Dies weist den erheblichen Nachteil auf, dass sie relativ bedingt gefaltet werden kann und daher für eine kleinschnitttaugliche Implantation in ein Auge nicht geeignet ist. Bei derartigen bekannten Linsen ist daher die Implantation in ein Auge, insbesondere in einen Kapselsack, mit relativ großen Schnitten verbunden, was auch wiederum nachteilig für den Patienten ist.

Darüber hinaus ist bei den beiden letztgenannten bekannten Intraokularlinsen der optische Teil durch das Teleskop gebildet. Dies hat wesentliche Nachteile im Hinblick auf das weitere Sehen des Patienten bei spezifischen Abbildungen des einfallenden Lichts. Darüber hinaus wird durch die Haltemechanik beziehungsweise die Träger in diesen bekannten Intraokularlinsen, die dieses Teleskop jeweils halten, der Sichtbereich ohnehin eingeschränkt, da diese erforderlichen Träger nicht zur optischen Abbildung beitragen.

### Darstellung der Erfindung

Es ist Aufgabe der vorliegenden Erfindung, eine Augenlinse zu schaffen, mittels welcher der Sehfehler AMD (Makuladegeneration) verbessert korrigiert werden kann.

Diese Aufgabe wird durch eine Augenlinse, welche die Merkmale nach Anspruch 1 aufweist, gelöst.

Eine erfindungsgemäße Intraokularlinse umfasst einen optischen Teil und einen daran anschließenden optisch unwirksamen haptischen Teil. Der optische Teil und somit ein optisch definiert wirkender Abbildungsteil weist ein optisch abbildendes Element und ein Teleskop auf. Dies bedeutet, dass hier zumindest zwei verschiedene optisch wirksame Komponenten vorhanden sind. Im Kontext der Erfindung ist es daher nicht derart zu verstehen, dass das Teleskop zugleich und vollständig auch das optisch abbildende Element ist.

Ein wesentlicher Gedanke der Erfindung ist darin zu sehen, dass das vollständige beziehungsweise gesamte Teleskop einstückig ausgebildet ist und in das optisch abbildende Element integriert ist. Dies bedeutet, dass somit das gesamte Teleskop auch einstückig mit dem optisch abbildenden Element ausgebildet ist und einstückig mit dem optisch abbildenden Element auch vorzugsweise hergestellt ist. Somit ist der optische Teil dieser Intraokularlinse mit zwei separaten optisch wirksamen, insbesondere optisch unterschiedlich wirksamen, Komponenten ausgebildet, die jedoch in einem gemeinsamen Verbund hergestellt und bereitgestellt sind. Diese einstückige Ausgestaltung ist jedoch derart, dass das optisch abbildende Element und das Teleskop nicht überlagert sind, sondern benachbart zueinander und somit nebeneinanderliegend ausgebildet sind und jeweils optisch individuell wirkend abbilden.

Diese erfindungsgemäße Intraokularlinse ist zur Sichtfeldanpassung und somit insbesondere zu einer Sichtfelderweiterung bei einer Makuladegeneration ausgebildet. Es wird durch diese spezifische Gestaltung des optischen Teils diese Sichtfeldanpassung im Vergleich zu Intraokularlinsen aus dem Stand der Technik wesentlich verbessert. Dies beispielsweise auch deswegen, da die Komplexität des Aufbaus reduziert ist, da das Teleskop und das optisch abbildende Element einstückig ausgebildet sind. Unerwünschte Positionstoleranzen zwischen einzelnen Linsenelementen eines Teleskops, wie sie im Stand der Technik auftreten, treten bei der erfindungsgemäßen Intraokularlinse nicht mehr auf, sodass gerade die Abbildungseigenschaft des Teleskops im besonderen Maße gegenüber den Ausführungen im Stand der Technik verbessert ist. Durch die zusätzliche Ausgestaltung mit dem optisch abbildenden Element wird über die optische Abbildungseigenschaft des Teleskops hinaus eine Zusatzfunktion im Hinblick auf die optische Wirkung des optischen Teils ergänzt und somit das gesamte optische Abbilden der Intraokularlinse verbessert, insbesondere bei einer Makuladegeneration. Indem erfindungsgemäß durch die Integration des gesamten Teleskops mit dem optisch abbildenden Element eine Positionsfixierung zwischen diesen Komponenten ausgebildet ist, können auch hier unerwünschten Positionstoleranzen und damit nachteilige Effekte auf die jeweiligen Abbildungseigenschaften der einzelnen Komponenten alleine als auch in Wirkverbindung vermieden werden.

Durch den einstückigen Aufbau von Teleskop und optisch abbildenden Element erreicht die Intraokularlinse eine relativ geringe Biegesteifigkeit, was für eine kleinschnitttaugliche Implantation in ein Auge besonders vorteilhaft ist.

Ein Teleskop ist insbesondere ein Element durch welches entfernte Gegenstände unter größerem Gesichtsfeld als mit freiem Auge und daher als näher gerückt gesehen werden. Die Brennweite der konvexen Seite des Teleskops ist größer als die Brennweite der konkaven Seite. Dadurch unterscheidet sich ein Teleskop auch wesentlich von einer konvex-konkav Linse.

Insbesondere ist vorgesehen, dass sich in einer Richtung senkrecht zu einer optischen Hauptachse der Intraokularlinse das Teleskop zentral mittig befindet und das optisch definiert abbildend wirkende Element als Ringelement ausgebildet ist und in dieser radialen Richtung unmittelbar an das Teleskop anschließend ausgebildet ist. In Umlaufrichtung um diese optische Hauptachse umgibt somit dann dieses optisch definiert abbildende Element das Teleskop umfangsseitig vollständig.

Das Teleskop ist ein konvex-konkav-Teleskop, welches als Einlinsensystem ausgebildet ist. Dies ist so orientiert, dass eine dem einfallenden Licht, insbesondere in einem im Auge implantierten Zustand der Intraokularlinse, zugewandte Vorderseite dieses Teleskops konvex gekrümmt ist und eine dem einfallenden Licht abgewandte Rückseite dieses Teleskops konkav gekrümmt ist. Durch diese Ausgestaltung wird eine besonders vorteilhafte Vergrößerungswirkung erzielt und somit eine Sichtfeldverbesserung bei einer Makuladegeneration erreicht. Durch diese Vergrößerung werden durch die Makuladegeneration beeinträchtigte Bereiche quasi nicht mehr abgebildet beziehungsweise weit an den Rand des Sichtfelds praktisch verschoben und somit ein verbesserter Sehvermögenseindruck beim Patienten erreicht.

Vorzugsweise ist vorgesehen, dass die konvex gekrümmte Vorderseite in einer Richtung senkrecht zur optischen Hauptachse der Intraokularlinse einen größeren Radius aufweist als die konkav gekrümmte Rückseite. Die oben genannten Vorteile werden dadurch nochmals begünstigt und ein spezifisches Abbildungsvermögen bei äußerst kompakter und insbesondere in Richtung der optischen Hauptachse sehr dünnen Intraokularlinse erreicht. Damit ist eine kleinschnitttaugliche Implantation in ein Auge besonders gut möglich.

Diese Ausgestaltung bedeutet auch, dass bezüglich der Längen, über welche sich die konkav gekrümmte Rückseite und die konvex gekrümmte Vorderseite in radialer Richtung zur optischen Hauptachse und somit senkrecht zur optischen Hauptachse erstrecken, eine Asymmetrie ausgebildet ist.

Vorzugsweise ist das Teleskop vollständig hohlraumfrei ausgebildet und somit quasi als massiver Körper gebildet. Das Teleskop weist damit zum einen eine hohe Eigenstabilität auf. Zum anderen können durch diese Ausgestaltung unerwünschte, aufgrund von unterschiedlichen Medien und somit unterschiedlichen Brechungsindizes auftretende Lichtstrahlablenkungen vermieden werden. Die Sichtfeldanpassung bei einer Makuladegeneration kann dadurch nochmals definierter und präziser erfolgen.

Vorzugsweise ist vorgesehen, dass das Teleskop in Richtung einer optischen Hauptachse des optischen Teils und somit auch der Intraokularlinse betrachtet sich beidseits über das radial an das Teleskop anschließende optisch abbildende beziehungsweise optisch wirkende Element hinaus erstreckt. Das Teleskop steht somit in dieser axialen Richtung nach vorne und nach hinten gegenüber dem optisch abbildenden Element über.

Vorzugsweise ist vorgesehen, dass eine dem einfallenden Licht zugewandte Vorderseite des Teleskops einen kleineren Radius aufweist als eine Vorderseite des optisch abbildenden Elements und an einem Übergang zwischen der Vorderseite des Teleskops und der Vorderseite des optisch abbildenden Elements ein Knick ausgebildet ist. Durch diesen nicht bündigen und somit unstetigen Konturenverlauf beziehungsweise mit einer gegenüber der Vorderseite des optisch abbildenden Elements als Ausbauchung erscheinende Wölbung der Vorderseite des Teleskops kann die Abbildungswirkung des Teleskops verbessert werden.

Vorzugsweise ist eine dem einfallenden Licht abgewandte, konkav gekrümmte Rückseite des Teleskops an eine Mantelwand des Teleskops mündend ausgebildet, und an einem Übergang eines Konturenverlaufs zwischen der Mantelwand und einer Rückseite des optisch abbildenden Elements ist ein Knick ausgebildet. Auch dadurch wird die Abbildungseigenschaft des Teleskops im Hinblick auf die Sichtfelderweiterung bei einer Makuladegeneration begünstigt. Diese jeweils in Richtung der optischen Hauptachse nach vorne und nach hinten überstehenden und erhabenen Formgebungen und Geometrien der Vorderseite und der Rückseite des Teleskops im Vergleich zu der Vorderseite und der Rückseite des direkt daran anschließenden optisch abbildenden Elements ist vorteilhaft, da somit auch ein sehr seitlicher Lichteinfall und/oder schräger Lichteinfall in das Teleskop ermöglicht ist.

Mit den spezifischen, jeweils mit einem Knick versehenen Übergängen beziehungsweise den dadurch ausgebildeten Angrenzungen der Vorderseiten und der Rückseiten des Teleskops einerseits und des optisch abbildend wirkenden Elements andererseits werden die gewünschten, dann auch unterschiedlichen Abbildungseigenschaften der separaten Komponenten verstärkt und die Präzision zur Sichtfeldanpassung bei einer Makuladegeneration verbessert.

Vorzugsweise ist das optisch abbildende Element eine Monofokallinse. Das optisch abbildende Element ist insbesondere eine Ringlinse.

In vorteilhafter Ausführung ist vorgesehen, dass die Intraokularlinse durch das Teleskop einen Vergrößerungsfaktor von zumindest 1,35, insbesondere größer 1,5, aufweist. Derartige, relativ große Vergrößerungswerte begünstigen die Sichtfeldanpassung bei einer Makuladegeneration wesentlich.

Zusätzlich oder anstatt dazu kann vorgesehen sein, dass die Intraokularlinse, insbesondere das Teleskop, eine entlang der optischen Hauptachse der Intraokularlinse bemessene Mittendicke von kleiner 2 mm aufweist. Eine derartige dünne Ausgestaltung der Intraokularlinse, insbesondere im optischen Teil auf der optischen Hauptachse, begünstigt eine kleinschnitttaugliche Implantation in ein Auge wesentlich. Eine derartig flachbauende Intraokularlinse kann sehr klein gefaltet werden und somit durch einen relativ kleinen Schnitt im Auge in das Auge eingeführt werden.

Vorzugsweise ist vorgesehen, dass das Material des optischen Elements und des Teleskops einen Brechungsindex größer 1,45 aufweist.

Bei vorteilhaften Ausführungen ist vorgesehen, dass, insbesondere durch die spezifische Geometrie des Teleskops und des optisch abbildenden Elements, beispielsweise mit einem Brechungsindexwert von 1,46, eine Vergrößerung beziehungsweise ein Vergrößerungsfaktor von zumindest 1,6 bei einer gleichzeitigen Mittendicke von maximal 2 mm erreicht ist. Mit größerem Brechungsindexwert kann bei reduzierter Mittendicke der gleiche Vergrößerungsfaktor erreicht werden. Eine Erhöhung des Vergrößerungsfaktors kann bei einer gleichen Mittendicke durch einen höheren Brechungsindex des Materials des Teleskops und des optisch wirkenden Elements erreicht werden. Entsprechend kann eine Reduzierung der Mittendicke bei gleichem Vergrößerungsfaktor durch Erhöhung des Brechungsindexwerts des Materials des Teleskops und des optisch wirkenden Elements erreicht werden. Vorzugsweise ist die Intraokularlinse so ausgebildet, dass bei einem Brechungsindexwert von 1,58 die Mittendicke um zumindest 45 Prozent reduziert bei aber gleichem Vergrößerungsfaktor im Vergleich zu einer Ausgestaltung mit einem Material mit einem Brechungsindexwert von 1,46 ausgebildet ist.

Insbesondere ist durch das zusätzliche optisch abbildende Element, insbesondere in Form eines das Teleskop radial außenliegend und umfangsseitig umgebenden Rings, auch ein peripheres Sehen mit der Intraokularlinse ermöglicht. Gerade dieses zusätzliche periphere Sehen ist dann auch im Hinblick auf die Abbildung des gesamten Sichtfelds gerade für Patienten mit Makuladegeneration besonders vorteilhaft.

In einer bevorzugten Ausführung ist vorgesehen, dass auf einer bei auf die Intraokularlinse einfallenden Licht dem Licht zugewandten Vorderseite des Teleskops eine diffraktive Struktur ausgebildet ist. Zusätzlich oder anstatt dazu kann vorgesehen sein, dass auf einer dem einfallenden Licht abgewandten Rückseite des Teleskops eine diffraktive Struktur ausgebildet ist. Eine diffraktive Struktur kann beispielsweise durch Fresnelzonen ausgebildet sein. Durch derartige diffraktive Strukturen ist in besonders vorteilhafter Weise eine Reduzierung der Mittendicke des optischen Teils erreicht, da auch das Teleskop in seiner Ausdehnung in Richtung der optischen Hauptachse kleiner ausgebildet werden kann. In besonders vorteilhafter Weise wird durch eine derartige diffraktive Struktur die Funktionalität der Intraokularlinse auch dahingehend erweitert, dass sie auch eine Tiefenschärfenlinse ist und dadurch die Tiefenschärfe erhöht ist. Eine derartige Intraokularlinse ermöglicht gerade für Patienten mit einer Makuladegeneration eine verbesserte Sichtfeldanpassung durch den Vergrößerungseffekt des Teleskops und verbessert zugleich eine scharfe Abbildung über einen größeren Bereich durch die Erhöhung der Tiefenschärfe.

Vorzugsweise ist das optisch abbildende Element so gestaltet, dass es eine Brechkraft zwischen 15 und 25 Dioptrien, vorzugsweise zwischen 17 und 22 Dioptrien, insbesondere 20 Dioptrien, aufweist.

In einer weiteren vorteilhaften Ausführung kann vorgesehen sein, dass eine Vorderseite und/oder eine Rückseite des optisch abbildenden Elements keine stetig sphärische oder asphärische Oberfläche ist, sondern zumindest eine Stufe in dem jeweiligen Oberflächenprofil ausgebildet ist. Insbesondere kann somit auch auf dem optisch abbildenden Element eine diffraktive Struktur ausgebildet sein. Insbesondere ist vorgesehen, dass dann, wenn sowohl auf der Vorderseite und/oder der Rückseite des optisch abbildenden Elements und auf der Vorderseite und/oder der Rückseite des Teleskops eine diffraktive Struktur ausgebildet ist, die diffraktiven Strukturen im Hinblick auf ihre Anzahl der diffraktiven Elemente, insbesondere in Form von Fresnelzonen, und/oder im Hinblick auf die geometrischen Ausgestaltungen dieser diffraktiven Zonen unterschiedlich ausgebildet sind.

Durch die erfindungsgemäße Intraokularlinse oder eine vorteilhafte Ausgestaltung davon können in sphärischen Wellen einfallende Lichtstrahlen in exakte ebenen Wellen umgewandelt werden oder umgekehrt. Es können auch exakte sphärische Wellen, ob konvergent oder divergent, in andere exakte sphärische Wellen, die auch konvergent oder divergent sein können, umgewandelt werden. Die Durchmesser von einfallenden Lichtstrahlen können durch die Intraokularlinse verändert werden. Durch das Teleskop kann eine an der Frontfläche beziehungsweise an der Vorderseite einfallende Welle, die beispielsweise eine ebene Welle ist, in eine konvergente sphärische Welle umgewandelt werden, und an der Rückseite des Teleskops kann diese konvergente sphärische Welle wiederum in eine ebene Welle umgewandelt werden. Der Durchmesser der resultierenden umgewandelten ebenen Welle ist dabei dann stets kleiner als der Durchmesser der eintretenden ebenen Welle.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen.

### Kurze Beschreibung der Zeichnungen

Ausführungsbeispiele der Erfindung werden nachfolgend anhand schematischer Zeichnungen näher erläutert. Es zeigen:
- Fig. 1a: eine perspektivische Darstellung eines ersten Ausführungsbeispiels einer erfindungsgemäßen Augenlinse;
- Fig. 1b: eine perspektivische Darstellung eines weiteren Ausführungsbeispiels einer erfindungsgemäßen Augenlinse;
- Fig. 2: eine Vertikalschnittdarstellung durch ein erstes Ausführungsbeispiel einer erfindungsgemäßen Intraokularlinse;
- Fig. 3: eine Vertikalschnittdarstellung durch ein zweites Ausführungsbeispiel einer erfindungsgemäßen Intraokularlinse;
- Fig. 4: eine Vertikalschnittdarstellung durch ein drittes Ausführungsbeispiel einer erfindungsgemäßen Intraokularlinse; und
- Fig. 5: eine Vertikalschnittdarstellung durch ein viertes Ausführungsbeispiel einer erfindungsgemäßen Intraokularlinse.

### Bevorzugte Ausführungen der Erfindung

In den Figuren werden gleiche oder funktionsgleiche Elemente mit den gleichen Bezugszeichen versehen.

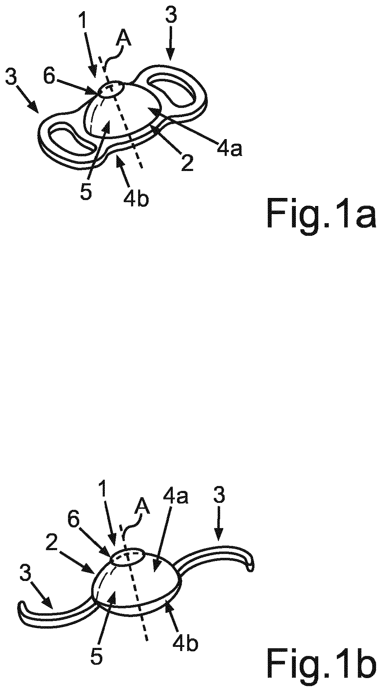

In Fig. 1 ist in einer perspektivischen Darstellung ein erstes Ausführungsbeispiel einer Intraokularlinse 1 gezeigt. Die Intraokularlinse 1 umfasst einen optischen Teil 2 und daran anschließend einen nur zum Halten der Intraokularlinse 1 im Auge ausgebildeten, optisch unwirksamen haptischen Teil bzw. eine Haptik 3. Die Intraokularlinse 1 ist faltbar und über einen Kleinschnitt in ein Auge einführbar. Der optische Teil 2, der wesentlich für die optische Abbildungseigenschaft der Intraokularlinse 1 ist, umfasst eine optische Hauptachse A, die senkrecht auf dem optischen Teil 2 steht. Der optische Teil 2 weist darüber hinaus in Richtung dieser optischen Hauptachse A betrachtet eine erste optische Fläche beziehungsweise Seite 4a, die eine Vorderseite sein kann, und gegenüberliegend eine zweite optische Fläche beziehungsweise Seite 4b, die eine Rückseite sein kann, auf. Die erste optische Seite 4a ist im implantierten Zustand der Intraokularlinse 1 im Auge der Hornhaut zugewandt, wohingegen die zweite optische Seite 4b dieser Hornhaut abgewandt ist.

In Fig. 1b ist in einer perspektivischen Darstellung ein weiteres Ausführungsbeispiel einer Intraokularlinse 1 gezeigt. Sie unterscheidet sich von der Ausführung in Fig. 1a durch die unterschiedliche Haptik 3. Mittels der Haptik 3 ist die Intraokularlinse 1 im Auge gehalten. Grundsätzlich können auch anderweitig geformte und ausgestaltete Haptiken 3 vorgesehen sein.

Wie in den Darstellungen in Fig. 1a und 1b schematisch zu erkennen ist, weisen die Intraokularlinsen 1 jeweils einen spezifisch ausgebildeten optischen Teil 2 auf. Der jeweilige optische Teil 2 umfasst in dem Zusammenhang insbesondere jeweils ein optisch abbildendes Element 5 und ein Teleskop 6. Das vollständige beziehungsweise gesamte Teleskop 6 ist in Fig. 1a und 1b jeweils einstückig und somit als einziges Teil ausgebildet. Dieses Teleskop 6 ist darüber hinaus in das optisch abbildende Element 5 integriert und somit mit diesem optisch abbildenden Element 5 auch einstückig ausgebildet und insbesondere auch einstückig hergestellt. Dies bedeutet, dass das Teleskop 6 zusammen mit dem optisch abbildenden Element 5 bei einem gemeinsamen Herstellungsverfahren ausgebildet wird.

Das einstückige optische Teil 2 kann in dem Zusammenhang aus einem Polymermaterial ausgebildet sein.

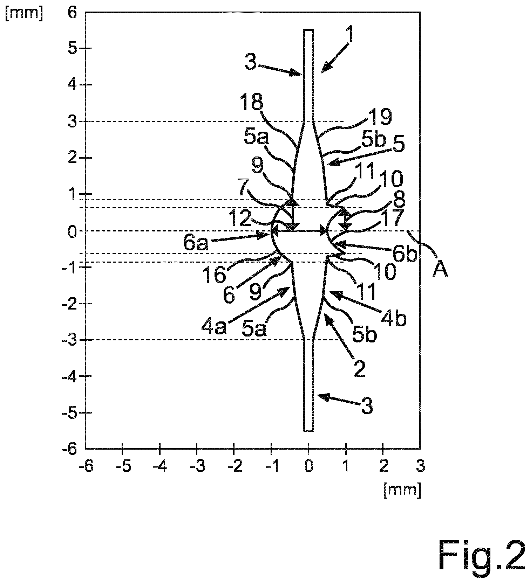

In Fig. 2 ist in einer schematischen Vertikalschnittdarstellung und somit in einer Schnittebene, die die optische Hauptachse A aufweist und durch die Haptik 3 und den optischen Teil 2 verläuft, ein erstes Ausführungsbeispiel einer Intraokularlinse 1 gezeigt. Die beispielhaften Maßangaben können auch anderweitig gestaltet sein, geben jedoch im Hinblick auf die Größenverhältnisse von Einzelteilen der Intraokularlinse 1 und insbesondere der Einzelteile des optischen Teils 2 Verhältniswerte an.

Bei der Darstellung in Fig. 2 ist zu erkennen, dass das Teleskop 6 insbesondere ein Galileo-Teleskop ist. Das einstückige Teleskop 6 ist symmetrisch um die Hauptachse A ausgebildet und sitzt zentral mittig in dem optischen Teil 2. In einer Richtung senkrecht zu der Hauptachse A betrachtet und somit in radialer Richtung zu der Hauptachse A grenzt dann das optisch abbildende Element 5 an, welches als Ring ausgebildet ist und das Teleskop 6 umlaufend um die Hauptachse A umgibt.

Im gezeigten Ausführungsbeispiel stellt die optische Seite 4a eine Vorderseite dar, was bedeutet, dass im implantierten Zustand der Intraokularlinse 1 im Auge, insbesondere im Kapselsack, diese optische Seite 4a der Hornhaut zugewandt ist. Demgegenüber ist im Ausführungsbeispiel dann die optische zweite Seite 4b der Hornhaut abgewandt.

Das optisch abbildende Element 5 ist im Ausführungsbeispiel eine Monofokallinse, die bikonvex ausgebildet ist. Dazu ist eine der Hornhaut im implantierten Zustand zugewandte beziehungsweise einem einfallenden Licht zugewandte Vorderseite 5a konvex gekrümmt, und eine dem einfallenden Licht abgewandte Rückseite 5b dieses optisch abbildenden Elements 5 ist ebenfalls konvex gekrümmt. Das optisch abbildende Element 5 ist vorzugsweise so ausgebildet, dass es eine Brechkraft von beispielsweise 20 Dioptrien aufweist.

Das Teleskop 6 ist, wie auch in Fig. 2 zu erkennen ist, als massive Komponente und somit ohne Hohlraum ausgebildet. Der gesamte Körper des Teleskops 6 wird somit mit dem Polymermaterial gefüllt. Das Teleskop 6 ist ein Einlinsensystem.

Wie zu erkennen ist, stellt das Teleskop 6 ein konvex-konkav-Teleskop dar. In dem Zusammenhang ist eine dem einfallenden Licht und somit auch im implantierten Zustand in das Auge eine der Hornhaut zugewandte Vorderseite 6a konvex gekrümmt ausgebildet, insbesondere vollständig konvex gekrümmt ausgebildet. Eine dem einfallenden Licht abgewandte und somit auch im implantierten Zustand der Intraokularlinse 1 der Hornhaut abgewandte Rückseite 6b des Teleskops 6 ist konkav gekrümmt, insbesondere vollständig konkav gekrümmt.

Wie darüber hinaus in Fig. 2 zu erkennen ist, weist die konvex gekrümmte Vorderseite 6a in einer Richtung senkrecht zur Hauptachse A einen größeren Radius 7 auf, als in dieser Richtung betrachtet die gekrümmte Rückseite 6b, die in dem Zusammenhang einen kleineren Radius 8 aufweist. Das Teleskop 6 ist somit von seiner Vorderseite 6a zu seiner Rückseite 6b hin - entlang der Hauptachse A - betrachtet verjüngt ausgebildet. Es stellt somit eine konusähnliche Form dar, wobei dieser Konus dann die spezifisch konvex geformte Vorderseite 6a und die spezifisch konkav geformte Rückseite 6b aufweist. Wie darüber hinaus zu erkennen ist, ist die Krümmung der Vorderseite 6a unterschiedlich, insbesondere kleiner, als die Krümmung der Rückseite 6b, da ein Radius 16 der Vorderseite größer ist, als ein Radius 17 der Rückseite 6b.

Darüber hinaus ist zu erkennen, dass der Radius 16 der konvexen Form der Vorderseite 6a kleiner ist als ein Radius 18 der konvexen Form der Vorderseite 5a des optisch abbildenden Elements 5 und ein Radius 19 der Rückseite 5b. Es ist daher ein eindeutiger, durch einen Knick 9 realisierter Übergang zwischen dem Teleskop 6 und dem optisch abbildenden Element 5 ausgebildet. Das Teleskop 6 wölbt sich an dieser vorderen ersten Seite 4a des optischen Teils 2 erhaben gegenüber der konvexen Wölbung der Vorderseite 5a des optisch abbildenden Elements 5 nach vorne beziehungsweise nach außen. Das Teleskop 6 erstreckt sich somit in Richtung der optischen Hauptachse A betrachtet nach vorne über das optisch abbildende Element 5 hinaus, und zwar mit seinem gesamten Ausmaß in Richtung senkrecht der Hauptachse A. Das bedeutet, dass die Vorderseite 6a, die an dem Knick 9 mit einem Rand bzw. einem Ende beginnt, bereits ab diesem Rand bzw. diesem Ende dann sich weiter nach vorne erstreckt als der diesbezüglich in Richtung der Hauptachse A betrachtet am weitesten vorne liegende Punkt der Vorderseite 5a.

Im Ausführungsbeispiel ist auch vorgesehen, dass sich das Teleskop 6 entlang der Hauptachse A betrachtet auch nach hinten gegenüber der konvex gekrümmten Rückseite 5b erhaben ausbildet und sich weiter nach außen bzw. nach hinten erstreckt. Auch hier ist an einer Anmündung beziehungsweise an einem Aneinanderangrenzen der Rückseite 5b und einer Mantelwand 10 des Teleskops 6 ein Knick 11 ausgebildet. Die Mantelwand 10 ist optisch inaktiv. Die konkav gekrümmte Rückseite 6b mündet nur an die Mantelwand 10 und ist lediglich über die Mantelwand 10 mit der Rückseite 5b verbunden.

Das Teleskop 6 ist insbesondere so ausgebildet, dass es einen Vergrößerungsfaktor von zumindest 1,35, insbesondere größer 1,5, aufweist und eine auf der optischen Hauptachse A bemessene Mittendicke 12 des optischen Teils 2, insbesondere des Teleskops 6, kleiner oder gleich 2 mm beträgt und das Material des optischen Teils 2 einen Brechungsindex von zumindest 1,45 oder größer aufweist.

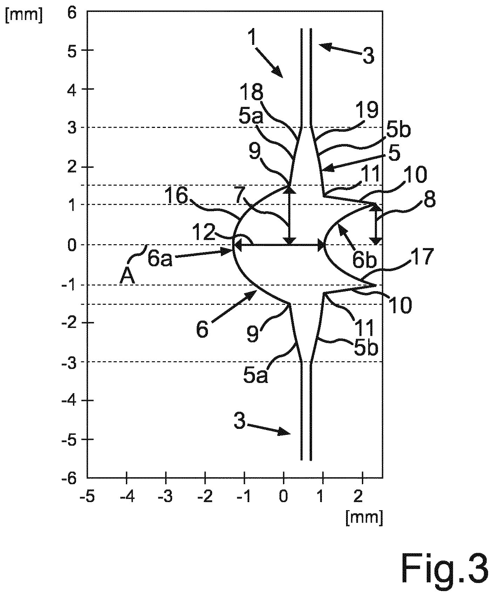

In Fig. 3 ist in einer weiteren Vertikalschnittdarstellung ein weiteres Ausführungsbeispiel einer Intraokularlinse 1 gezeigt. Diese weist im Unterschied zur Darstellung in Fig. 2 lediglich unterschiedliche Dimensionierungen des Teleskops 6 im Vergleich zum Element 5 auf.

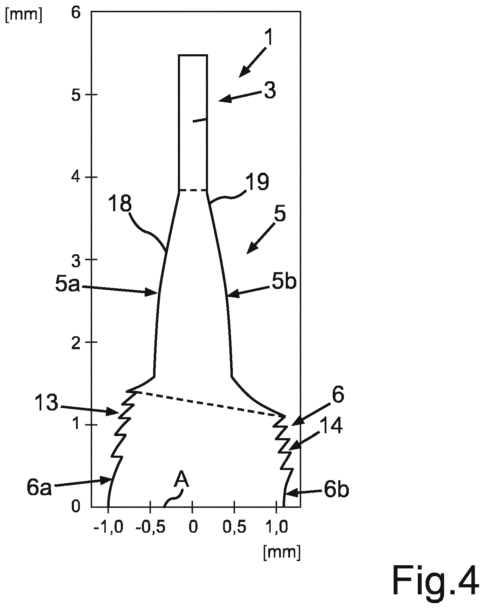

In Fig. 4 ist in einer weiteren Vertikalschnittdarstellung ein Ausführungsbeispiel einer Intraokularlinse 1 gezeigt, wobei hier lediglich ein oberer Teil oberhalb der optischen Hauptachse A dargestellt ist. Wie hier zu erkennen ist, ist auf die Oberfläche der konvex gekrümmten Vorderseite 6a bereichsweise eine diffraktive Struktur 13 aufgebracht, die im Ausführungsbeispiel mehrere Fresnelzonen sind. Darüber hinaus ist im Ausführungsbeispiel vorgesehen, dass auch auf der konkav gekrümmten Rückseite 6b des Teleskops 6 zumindest teilweise auf dieser konkav gekrümmten Oberfläche eine weitere diffraktive Struktur 14 aufgebracht ist, die ebenfalls vorzugsweise durch mehrere Fresnelzonen gebildet ist.

Es kann auch vorgesehen sein, dass nur auf der Vorderseite 6a oder nur auf der Rückseite 6b jeweils eine diffraktive Struktur 13 beziehungsweise 14 aufgebracht ist. Die diffraktiven Strukturen 13 und/oder 14 sind in radialer Richtung an einem äußeren, an das optisch abbildende Element 5 angrenzenden Bereich des Teleskops 6 ausgebildet und somit im Beispiel nur bereichsweise auf der Vorderseite 6a und/oder der Rückseite 6b ausgebildet.

Insbesondere kann vorgesehen sein, dass die diffraktiven Strukturen 13 und 14 unterschiedlich sind. Dies bedeutet, dass die Anzahl der diffraktiven Zonen unterschiedlich sein kann und/oder die geometrischen Ausgestaltungen der diffraktiven Zonen unterschiedlich sein können.

Bei der Darstellung in Fig. 4 ist auch wiederum in vertikaler und in horizontaler Richtung eine Skalierung angegeben, und auch hier sind die Werte im Hinblick auf die Größenverhältnisse der einzelnen Teile der Intraokularlinse 1 insbesondere als maßstabsgetreu zu verstehen.

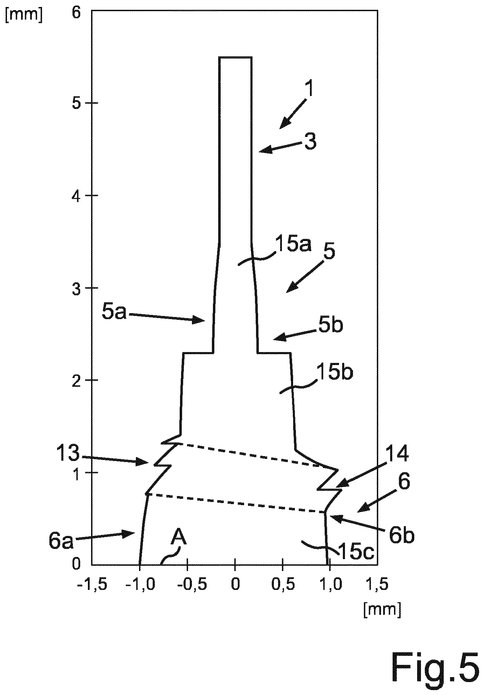

In Fig. 5 ist ein weiteres Ausführungsbeispiel einer Intraokularlinse 1 in einer Vertikalschnittdarstellung gezeigt, wobei auch hier in entsprechender Weise wie in Fig. 4 lediglich eine Darstellung oberhalb einer optischen Hauptachse A gezeigt ist. Bei dieser Ausführung ist vorgesehen, dass die Vorderseite 5a und auch die Rückseite 5b des optisch abbildenden Elements 5 jeweils eine diffraktive Struktur aufweisen und in dem Zusammenhang gestuft ausgebildet sind und somit nicht stetig konvex geformt sind.

Es kann auch vorgesehen sein, dass nur die Vorderseite 5a oder nur die Rückseite 5b eine derartige diffraktive Struktur aufweisen.

Durch diese Ausgestaltung werden monofokale Bereiche 15a, 15b und 15c gebildet.

## Patentansprüche

1. Intraokularlinse (1) mit einem optischen Teil (2) und einem daran anschließenden haptischen Teil (3), wobei der optische Teil (2) ein optisch abbildendes Element (5) und ein Teleskop (6) aufweist,
das gesamte Teleskop (6) einstückig ausgebildet ist und in das optisch abbildende Element (5) integriert ist, wobei das Teleskop ein als Einlinsensystem ausgebildetes konvex-konkav Teleskop (6) ist, und eine bei auf die Intraokularlinse (1) einfallendem Licht dem einfallenden Licht zugewandte Vorderseite (6a) konvex gekrümmt ist und eine dem einfallenden Licht abgewandte Rückseite (6b) konkav gekrümmt ist,
**dadurch gekennzeichnet, dass**
das Teleskop auch einstückig mit dem optisch abbildenden Element ausgebildet ist.

2. Intraokularlinse (1) nach Anspruch 1, wobei die konvex gekrümmte Vorderseite (6a) in einer Richtung senkrecht zur optischen Hauptachse (A) der Intraokularlinse (1) einen größeren Radius (7) aufweist, als die konkav gekrümmte Rückseite (6b).

3. Intraokularlinse (1) nach einem der vorhergehenden Ansprüche, wobei das Teleskop (6) hohlraumfrei ausgebildet ist.

4. Intraokularlinse (1) nach einem der vorhergehenden Ansprüche, wobei das Teleskop (6) in Richtung einer optischen Hauptachse (A) des optischen Teils (2) betrachtet sich beidseits über das radial an das Teleskop (6) anschließende optisch abbildende Element (5) hinaus erstreckt.

5. Intraokularlinse (1) nach einem der vorhergehenden Ansprüche, wobei eine bei auf die Intraokularlinse (1) einfallendem Licht dem einfallenden Licht zugewandte Vorderseite (6a) des Teleskops (6) einen kleineren Radius (16) aufweist als eine Vorderseite (5a) des optisch abbildenden Elements (5) und an einem Konturenübergang zwischen der Vorderseite (6a) des Teleskops (6) und der Vorderseite (5a) des optisch abbildenden Elements (5) ein Knick (9) ausgebildet ist.

6. Intraokularlinse (1) nach einem der vorhergehenden Ansprüche, wobei eine bei auf die Intraokularlinse (1) einfallendem Licht dem einfallenden Licht abgewandte, konkav gekrümmte Rückseite (6b) des Teleskops (6) an eine Mantelwand (10) des Teleskops (6) mündet und an einem Konturenübergang zwischen der Mantelwand (10) und einer Rückseite (5b) des optisch abbildenden Elements (5) ein Knick (11) ausgebildet ist.

7. Intraokularlinse (1) nach einem der vorhergehenden Ansprüche, wobei das optisch abbildende Element (5) eine Monofokallinse ist, insbesondere eine monofokale Ringlinse ist.

8. Intraokularlinse (1) nach einem der vorhergehenden Ansprüche, wobei die Intraokularlinse (1) durch das Teleskop (6) einen Vergrößerungsfaktor von zumindest 1,35, insbesondere größer 1,5, aufweist.

9. Intraokularlinse (1) nach einem der vorhergehenden Ansprüche, wobei die Intraokularlinse (1), insbesondere das Teleskop (6), eine entlang der optischen Hauptachse (A) der Intraokularlinse (1) bemessene Mittendicke (12) von kleiner 2mm aufweist.

10. Intraokularlinse (1) nach einem der vorhergehenden Ansprüche, wobei auf einer bei auf die Intraokularlinse (1) einfallendem Licht dem einfallenden Licht zugewandten Vorderseite (6a) des Teleskops (6) eine diffraktive Struktur (13) und/oder auf einer dem einfallenden Licht abgewandten Rückseite (6b) des Teleskops (6) eine diffraktive Struktur (14) ausgebildet ist.

## Claims

1. Intraocular lens (1) comprising an optical part (2) and an adjoining haptic part (3), wherein the optical part (2) comprises an optically imaging element (5) and a telescope (6),
the entire telescope (6) has an integral embodiment and is integrated into the optically imaging element (5), wherein the telescope is a convex-concave telescope (6) embodied as a single lens system, and a front side (6a) facing the incident light in the case of light incident on the intraocular lens (1) has convex curvature and a rear side (6b) facing away from the incident light has concave curvature,
**characterized in that**
the telescope also has an integral embodiment with the optically imaging element.

2. Intraocular lens (1) according to Claim 1,
wherein
the convexly curved front side (6a) has a greater radius (7) in a direction perpendicular to the optical main axis (A) of the intraocular lens (1) than the concavely curved rear side (6b).

3. Intraocular lens (1) according to either of of the preceding claims,
wherein
the telescope (6) has a cavity-free embodiment.

4. Intraocular lens (1) according to any one of the preceding claims,
wherein
the telescope (6), on both sides, extends beyond the optically imaging element (5) radially adjoining the telescope (6) when viewed in a direction of an optical main axis (A) of the optical part (2).

5. Intraocular lens (1) according to any one of the preceding claims,
wherein
a front side (6a) of the telescope (6) facing the incident light in the case of light incident on the intraocular lens (1) has a smaller radius (16) than a front side (5a) of the optically imaging element (5) and a kink (9) is formed at a contour transition between the front side (6a) of the telescope (6) and the front side (5a) of the optically imaging element (5).

6. Intraocular lens (1) according to any one of the preceding claims,
wherein
a concavely curved rear side (6b) of the telescope (6) facing away from the incident light in the case of light incident on the intraocular lens (1) opens into a lateral wall (10) of the telescope (6) and a kink (11) is formed at a contour transition between the lateral wall (10) and a rear side (5b) of the optically imaging element (5).

7. Intraocular lens (1) according to any one of the preceding claims,
wherein
the optically imaging element (5) is a monofocal lens, in particular a monofocal ring lens.

8. Intraocular lens (1) according to any one of the preceding claims,
wherein
the intraocular lens (1) has a magnification factor of at least 1.35, in particular greater than 1.5, through the telescope (6).

9. Intraocular lens (1) according to any one of the preceding claims,
wherein
the intraocular lens (1), in particular the telescope (6), has a central thickness (12) of less than 2 mm as measured along the optical main axis (A) of the intraocular lens (1).

10. Intraocular lens (1) according to any one of the preceding claims,
wherein
a diffractive structure (13) is embodied on a front side (6a) of the telescope (6) facing the incident light in the case of light incident on the intraocular lens (1) and/or a diffractive structure (14) is embodied on a rear side (6b) of the telescope (6) facing away from the incident light.

## Revendications

1. Lentille intraoculaire (1) comprenant une partie optique (2) et une partie haptique (3) raccordée à celle-ci, la partie optique (2) comportant un élément de reproduction optique (5) et un télescope (6),
le télescope entier (6) étant formé d'une seule pièce et étant intégré dans l'élément de reproduction optique (5), le télescope étant un télescope convexe-concave (6) conçu comme un système à une seule lentille, et une face avant (6a), laquelle, lorsqu'une lumière est incidente à la lentille intraoculaire (1), est dirigée vers la lumière incidente, étant incurvée de manière convexe et une face arrière (6b), opposée à la lumière incidente, étant incurvée de manière concave,
**caractérisée en ce que**
le télescope est également conçu d'une seule pièce avec l'élément de reproduction optique.

2. Lentille intraoculaire (1) selon la revendication 1,
la face avant (6a), incurvée de manière convexe, ayant un rayon (7) qui est supérieur à celui de la face arrière (6b), incurvée de manière concave, dans une direction perpendiculaire à l'axe optique principal (A) de la lentille intraoculaire (1).

3. Lentille intraoculaire (1) selon l'une des revendications précédentes,
le télescope (6) étant conçu de manière à être dépourvu de cavité.

4. Lentille intraoculaire (1) selon l'une des revendications précédentes,
le télescope (6), lorsqu'on l'observe en direction d'un axe optique principal (A) de la partie optique (2), s'étendant des deux côtés au-delà de l'élément de reproduction optique (5) se raccordant radialement au télescope (6) .

5. Lentille intraoculaire (1) selon l'une des revendications précédentes,
une face avant (6a) du télescope (6), laquelle, lorsque la lumière est incidente à la lentille intraoculaire (1), est dirigée vers la lumière incidente, ayant un rayon (16) qui est inférieur à celui d'une face avant (5a) de l'élément de reproduction optique (5) et un coude (9) étant formé au niveau d'une transition de contour entre la face avant (6a) du télescope (6) et la face avant (5a) de l'élément de reproduction optique (5) .

6. Lentille intraoculaire (1) selon l'une des revendications précédentes, une face arrière (6b), incurvée concave, du télescope (6), laquelle, lorsque la lumière est incidente à la lentille intraoculaire (1), est opposée à la lumière incidente, donnant sur une paroi d'enveloppe (10) du télescope (6) et un coude (11) étant formé au niveau d'une transition de contour entre la paroi d'enveloppe (10) et une face arrière (5b) de l'élément de reproduction optique (5).

7. Lentille intraoculaire (1) selon l'une des revendications précédentes, l'élément de reproduction optique (5) étant une lentille monofocale, en particulier une lentille monofocale annulaire.

8. Lentille intraoculaire (1) selon l'une des revendications précédentes, la lentille intraoculaire (1) présentant à travers le télescope (6) un facteur de grossissement d'au moins 1,35, en particulier de plus de 1,5.

9. Lentille intraoculaire (1) selon l'une des revendications précédentes, la lentille intraoculaire (1), en particulier le télescope (6), ayant une épaisseur centrale (12) inférieure à 2 mm, mesurée suivant l'axe optique principal (A) de la lentille intraoculaire (1).

10. Lentille intraoculaire (1) selon l'une des revendications précédentes, une structure diffractive (13) étant formée sur une face avant (6a) du télescope (6) qui, lorsqu'une lumière est incidente à la lentille intraoculaire (1), est dirigée vers la lumière incidente et/ou une structure diffractive (14) étant formée sur la face arrière (6b) du télescope (6) qui est opposée à la lumière incidente.
